# EUROPEAN PATENT APPLICATION

(11) **EP 4 527 440 A1**
(43) Date of publication of application: **26.03.2025**
(21) Application number: 23807609.5
(22) Date of filing: 15.05.2023
(51) Int. Cl.: A61M 25/00, A61M 25/14, A61B 17/22

(54) **THROMBUS SUCTION CATHETER**

(30) Priority: 20.05.2022 JP 2022083095
(71) Applicant: Nipro Corporation, Osaka 566-8510 (JP)
(72) Inventor: AKIHAMA, Hidenobu, Osaka-shi, Osaka 531-8510 (JP)
(74) Representative: Slingsby Partners LLP
(86) International application number: PCT/JP2023/018135
(87) International publication number: WO 2023/224007

(57) **Abstract**

A thrombus suction catheter 10 includes: a suction tube 12 having a suction lumen 18 that suctions a thrombus within a blood vessel; a guide wire insertion tube 14 having a guide wire lumen 24 into which a guide wire 56 is inserted; and a distal tip 16 attached to distal ends of the suction tube 12 and the guide wire insertion tube 14 while having a distal lumen 34 communicating with the suction lumen 18 of the suction tube 12. The distal tip 16 is provided with a tube mounting groove 38 extending in an axial direction on an outer peripheral surface thereof, and the guide wire insertion tube 14 separate from the distal tip 16 is inserted into the tube mounting groove 38 and fixed to the distal tip 16.

## Description

### TECHNICAL FIELD

This invention relates to a thrombus suction catheter used for suction removal of a thrombus in a blood vessel.

### BACKGROUND ART

Peripheral arterial disease (PAD) caused by insufficient blood flow has been known from the past, and if it progresses, it may cause intermittent claudication, ulcers, necrosis, etc., so treatment may be required. For example, in the case of peripheral arterial disease caused by the deterioration of blood flow due to arteriosclerosis, treatment to resolve thrombotic lesions by thrombus retrieval therapy to retrieve and remove thrombi in blood vessels has also been proposed. One method of retrieving thrombi in blood vessels is suction removal of thrombi using a thrombus suction catheter.

As disclosed in Japanese Unexamined Patent Publication No. JP-A-2013-126573 (Patent Document 1) , for example, a thrombus suction catheter is provided with a suction lumen for suctioning a thrombus, and is inserted into a blood vessel percutaneously. By suctioning blood containing a thrombus into the suction lumen, a thrombus is removed from the blood vessel.

### BACKGROUND ART DOCUMENT

### PATENT DOCUMENT

Patent Document 1: JP-A-2013-126573

### SUMMARY OF THE INVENTION

### PROBLEM THE INVENTION ATTEMPTS TO SOLVE

Meanwhile, the thrombus suction catheter of Patent Document 1 has a shape with its distal end cut at a bevel, and the distal side opening of the suction lumen opens obliquely toward the front (the distal side). By making the distal side opening of the suction lumen oblique in this way, it is expected to sufficiently obtain the opening area and adjust the direction of action of the suction force, etc.

However, the structure of Patent Document 1 is limited to a shape in which the distal end of the thrombus suction catheter is cut at a bevel, so that the design freedom of the distal end of the catheter, which greatly affects the direction of action of the suction force and the ease of insertion into blood vessels, etc., is greatly limited. In particular, if the opening direction of the suction lumen is simply made oblique, although a thrombus in front is suctioned, it is difficult to effectively apply suction force to a lateral thrombus adhered to the blood vessel wall, for example, and there is a possibility that efficient suction removal of thrombi would not be achieved.

It is therefore one object of the present invention to provide a thrombus suction catheter of novel structure which is able to allow its distal end shape to be designed with a large degree of freedom.

### MEANS FOR SOLVING THE PROBLEM

Hereinafter, preferred embodiments for grasping the present invention will be described. However, each preferred embodiment described below is exemplary and can be appropriately combined with each other. Besides, a plurality of elements described in each preferred embodiment can be recognized and adopted as independently as possible, or can also be appropriately combined with any element described in other preferred embodiments. By so doing, in the present invention, various other preferred embodiments can be realized without being limited to those described below.

The present inventor considered that a large degree of freedom in the shape of the distal end of the catheter could be obtained by configuring the distal end of the thrombus suction catheter with a distal tip separate from the tube portion. However, when the inventor conducted prototyping and examination, he found that, for example, if the distal tip was made as a molded product such as an injection-molded product, although the degree of freedom in the shape of the distal end of the catheter would increase, it would be difficult to mold a small-diameter lumen through which a guide wire could be inserted, and this would be a factor that would limit the shape of the distal tip. The inventor then came up with the present invention based on such new findings.

A first preferred embodiment provides a thrombus suction catheter comprising: a suction tube including a suction lumen configured to suction a thrombus within a blood vessel; a guide wire insertion tube including a guide wire lumen configured to allow a guide wire to be inserted; and a distal tip attached to distal ends of the suction tube and the guide wire insertion tube, the distal tip including a distal lumen communicating with the suction lumen of the suction tube, wherein a tube mounting groove extending in an axial direction is provided on an outer peripheral surface of the distal tip, and the guide wire insertion tube separate from the distal tip is inserted into the tube mounting groove and fixed to the distal tip.

According to the thrombus suction catheter structured following the present preferred embodiment, the catheter distal end is constituted by the distal tip that is separate from the suction tube and the guide wire insertion tube. Thus, for example, by making the distal tip as a molded product such as an injection-molded product, a large degree of freedom of the shape of the catheter distal end can be obtained. Furthermore, because the distal tip is separate from the guide wire insertion tube, the shape and the structure of the distal tip can be designed with a larger freedom without the need of considering the formability of a small-diameter guide wire lumen. The ability to design the distal tip with a large degree of freedom also makes it possible, for example, to form the distal opening of the distal lumen connected to the suction lumen so as to open to the lateral side of the distal tip, etc., thereby setting the direction of action of the suction force or the like with a large degree of freedom.

A second preferred embodiment provides the thrombus suction catheter according to the first preferred embodiment, wherein the distal lumen includes a suction opening part opening to a lateral side on the outer peripheral surface of the distal tip, and an obstruction wall face obstructing a distal side of the distal lumen is provided on the distal side of the suction opening part of the distal tip, and the tube mounting groove is arranged at a position away from the suction opening part in a circumferential direction of the distal tip.

According to the thrombus suction catheter structured following the present preferred embodiment, the suction opening part of the distal lumen does not open toward the distal end of the distal tip, but opens to the lateral side of the distal tip. This allows the suction force to exert efficiently on the lateral side of the distal tip, thereby efficiently suctioning and removing the thrombus adhered to the wall inner surface of the blood vessel wall, for example.

The suction opening part opens at the position away from the tube mounting groove in the circumferential direction. This makes it possible to efficiently ensure the opening area of the suction opening part without being covered by the guide wire insertion tube attached to the distal tip, and to easily bring the suction opening part closer to the blood vessel wall or the like.

A third preferred embodiment provides the thrombus suction catheter according to the second preferred embodiment, wherein a distal end portion of the distal tip comprises a small-diameter part including a tapered part, while a proximal end portion of the distal tip comprises a large-diameter part having a circular cylinder shape with a diameter larger than that of the small-diameter part, and the suction opening part opens on the large-diameter part.

According to the thrombus suction catheter structured following the present preferred embodiment, the distal end portion of the distal tip comprises the small-diameter part, which provides excellent ease of insertion into the blood vessel, etc., and in particular, facilitates insertion into the stenosis portion of the blood vessel. Besides, the proximal end portion of the distal tip comprises the large-diameter part, which makes it possible to ensure a large cross-sectional area of the suction lumen and a large opening area of the suction opening part, thereby efficiently retrieving the thrombi.

A fourth preferred embodiment provides the thrombus suction catheter according to the second or third preferred embodiment, wherein the distal tip is formed of a resin material mixed with an X-ray opaque material, and the distal tip includes an X-ray transmission hole penetrating a bottom wall of the tube mounting groove, the X-ray transmission hole being formed at a position that overlaps with the suction opening part in a diametrical direction of the distal tip.

According to the thrombus suction catheter structured following the present preferred embodiment, the distal tip enables visibility under the X-ray fluoroscopy to be reliably obtained. **In** particular, by adopting the distal tip made of a resin material mixed with an X-ray opaque material, it may be possible to reliably obtain visibility at a lower cost than when using a ring marker made of a precious metal, etc.

Additionally, when the distal tip is visually observed in the direction of opening of the suction opening part under the X-ray fluoroscopy, the X-ray transmissivity is increased in the portion where the X-ray transmission hole and the suction opening part overlap with each other, so that the imaging of the portion where the X-ray transmission hole and the suction opening part overlap with each other is faint. Therefore, even under the X-ray fluoroscopy with low resolution, it is possible to relatively easily specify the direction of opening of the suction opening part based on the density of the imaging.

A fifth preferred embodiment provides the thrombus suction catheter according to any one of the second through fourth preferred embodiments, wherein a distal side wall face of the suction opening part has an inclination angle with respect to the axial direction of the distal tip larger than that of a proximal side wall face of the suction opening part.

According to the thrombus suction catheter structured following the present preferred embodiment, the inclination angle of the distal side wall face of the suction opening part is made relatively large. Thus, for example, when using the distal side wall face of the suction opening part to scrape off the thrombus adhered to the wall inner surface of the blood vessel, it is possible to efficiently scrape off the thrombus, thereby enhancing the performance of thrombus retrieval. Besides, the inclination angle of the proximal side wall face of the suction opening part is made relatively small. Thus, when the thrombus suction catheter is inserted into a blood vessel or the like and pushed to the distal side, the proximal side wall face of the suction opening part is less likely to get caught on the blood vessel wall or the like, and smooth insertion is possible.

A sixth preferred embodiment provides the thrombus suction catheter according to any one of the first through fifth preferred embodiments, further comprising a control mechanism configured to induce curving deformation of the distal ends of the suction tube and the guide wire insertion tube by exerting operating force from an outside.

According to the thrombus suction catheter structured following the present preferred embodiment, by using the control mechanism to induce curving deformation of the distal end of the suction tube, for example, it is possible to bring the thrombus suction port, which is connected to the suction lumen, closer to the thrombus to efficiently suction and remove the thrombus.

### EFFECT OF THE INVENTION

According to the present invention, it is possible to design the distal end shape of the thrombus suction catheter with a large degree of freedom.

### BRIEF DESCRIPTION OF THE DRAWINGS

FIG. 1 is a right side view showing a thrombus suction catheter according to a first practical embodiment of the present invention.
FIG. 2 is a plan view of the thrombus suction catheter shown in FIG. 1.
FIG. 3 is a cross sectional view taken along line 3-3 of FIG. 2.
FIG. 4 is a cross sectional view taken along line 4-4 of FIG. 3.
FIG. 5 is a cross sectional view taken along line 5-5 of FIG. 3.
FIG. 6 is a cross sectional view taken along line 6-6 of FIG. 3.
FIG. 7 is a cross sectional view taken along line 7-7 of FIG. 3.
FIG. 8 is a cross sectional view taken along line 8-8 of FIG. 3.
FIG. 9 is a perspective view of a distal tip constituting the thrombus suction catheter shown in FIG. 1.
FIG. 10 is a perspective view of the distal tip shown in FIG. 9 at another angle.
FIG. 11A is an X-ray photograph of the thrombus suction catheter shown in FIG. 1 taken from above.
FIG. 11B is an X-ray photograph of the thrombus suction catheter shown in FIG. 1 taken from the right.
FIG. 12 is a cross sectional view showing a thrombus suction catheter according to a second practical embodiment of the present invention.

### EMBODIMENTS FOR CARRYING OUT THE INVENTION

Practical embodiments of the present invention will be described below in reference to the drawings.

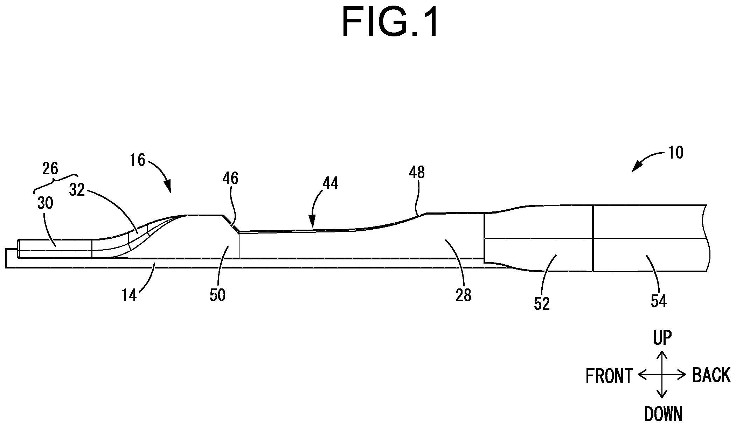

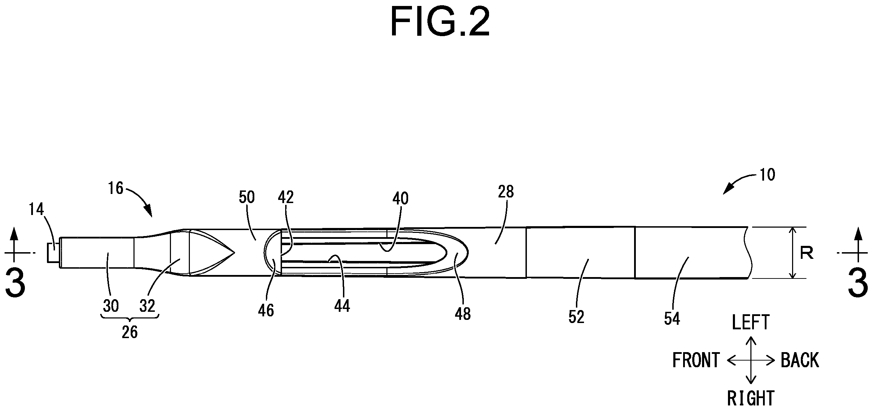

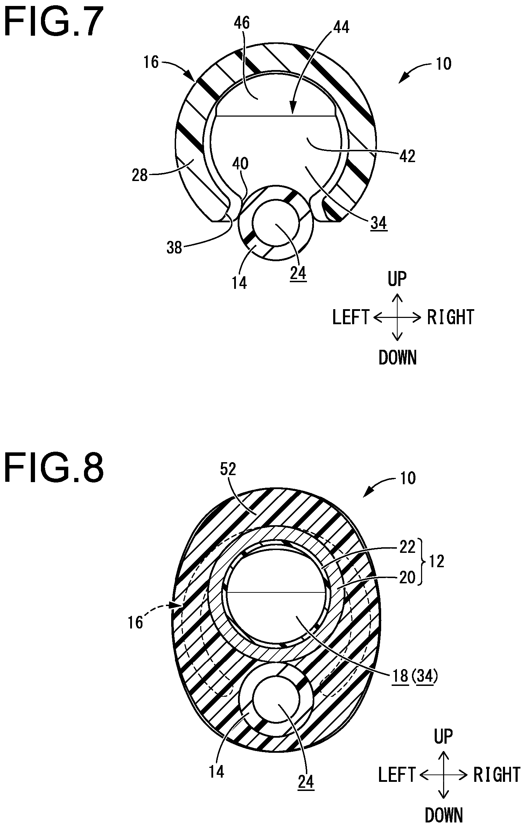

FIGS. 1 to 8 show a thrombus suction catheter 10 according to a first practical embodiment of the present invention. The thrombus suction catheter 10 is a catheter used in thrombus suction therapy to suction and remove thrombi from blood vessels, and is particularly suitable for removing thrombi from blood vessels in the lower limbs. The thrombus suction catheter 10 has a structure in which a distal tip 16 is attached to the distal ends of a suction tube 12 and a guide wire insertion tube 14. In the following description, as a general rule, the vertical direction refers to the vertical direction in FIG. 1. In addition, as a general rule, the front-back direction refers to the left-right direction in FIG. 1, and the left-right direction refers to the vertical direction in FIG. 2.

The suction tube 12 is a hollow body with an approximately circular cross-section, and the lumen that penetrates the suction tube 12 in the axial direction comprises a suction lumen 18. The suction tube 12 of the present practical embodiment is provided with a two-layered peripheral wall including a braided layer 20 comprising a braided body formed by weaving metal wire such as stainless steel, and an inner layer 22 comprising a low-friction material such as fluororesin. By including the braided layer 20, the suction tube 12 has flexibility while reliably obtaining pushability in the axial direction. In addition, the inner circumferential surface of the braided layer 20 is covered by the low-friction inner layer 22, so that the flow resistance of the suction lumen 18 is reduced. Here, the peripheral wall of the suction tube 12 may have a multi-layer structure of three or more layers, or a single-layer structure of only one layer. Besides, the braided layer 20 is not essential, and for example, the peripheral wall of the suction tube 12 may have a multi-layer structure made of multiple types of resin materials with different properties, or a reinforcing layer of spiral metal wire may be provided in place of or in addition to the braided layer 20.

The guide wire insertion tube 14 is a hollow body with an approximately circular cross-section, and the lumen that penetrates the guide wire insertion tube 14 in the axial direction comprises a guide wire lumen 24. The guide wire insertion tube 14 is formed of synthetic resin or the like and has flexibility. The guide wire insertion tube 14 has an inner diameter dimension and an outer diameter dimension that are smaller than those of the suction tube 12. The guide wire insertion tube 14 does not have a braided body like the suction tube 12, and is more flexible than the suction tube 12. The guide wire insertion tube 14 of the present practical embodiment has a single-layer peripheral wall, but it may also have a multi-layer peripheral wall, for example, comprising two or more types of resin or the like. It is desirable that the guide wire insertion tube 14 have a higher X-ray transmissivity than that of the distal tip 16, and is preferably made of a material that does not contain an X-ray opaque material.

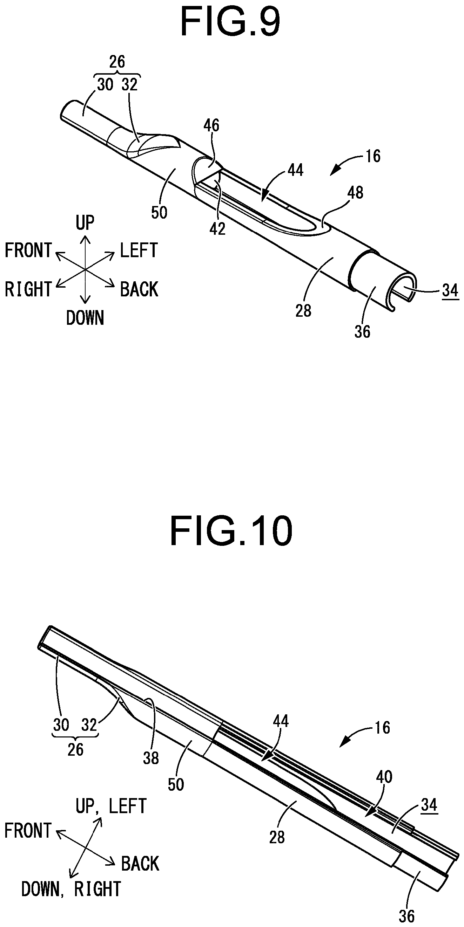

The distal tip 16 has both the flexibility to the extent that it can follow the curve of the blood vessel and the deformation rigidity to the extent that a distal lumen 34 (described later) is not collapsed by suction force (negative pressure). The distal tip 16 is formed of an X-ray opaque resin in which, for example, a resin material such as polyethylene is mixed with an X-ray opaque material such as bismuth oxide. As shown in FIGS. 9 and 10, the distal tip 16 integrally includes a small-diameter part 26 constituting the distal end portion and a large-diameter part 28 constituting the portion on the proximal side of the small-diameter part 26. The distal tip 16, the suction tube 12, and the guide wire insertion tube 14 are mutually separate components.

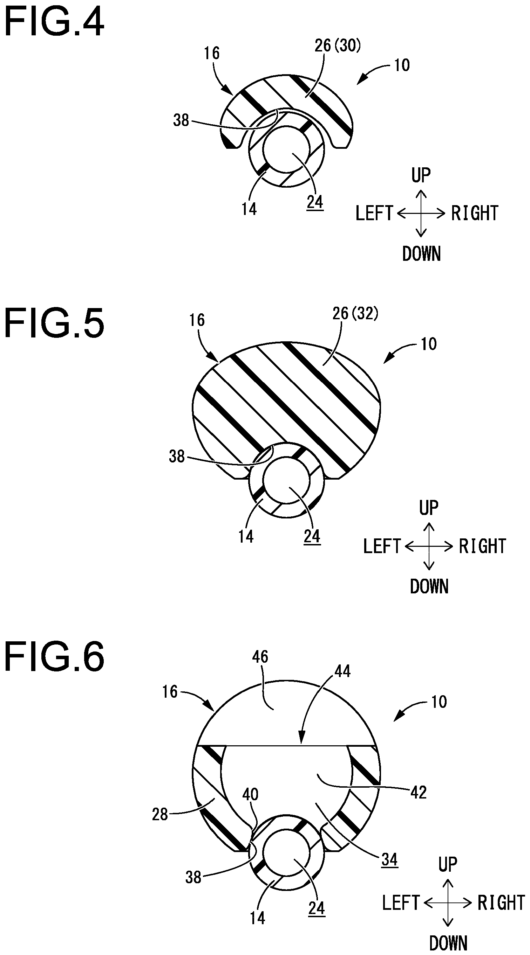

In the small-diameter part 26, the distal end portion comprises a semi-cylindrical part 30 that extends in the axial direction with an approximately constant outer diameter dimension, and the proximal end portion comprises a tapered part 32 that decreases in diameter toward the distal end. The outer peripheral surface of the tapered part 32 is constituted by a smooth curved surface, and is smoothly continuous with the semi-cylindrical part 30. As shown in FIG. 5, it is desirable that the small-diameter part 26 be flattened in the vertical direction, at least in the tapered part 32.

The large-diameter part 28 has an approximately circular cylinder shape with a diameter larger than that of the small-diameter part 26, and includes a distal lumen 34 inside that extends in the axial direction and opens at the proximal face. In the present practical embodiment, the proximal end of the large-diameter part 28 is provided with a thin-walled connection part 36 whose outer diameter dimension is decreased in a stepped manner. For example, in the case where the distal tip 16 is obtained by molding, it is desirable to set an extraction taper in the large-diameter part 28 to facilitate removal of the mold when the molded article is demolded in the axial direction, and the large-diameter part 28 of the present practical embodiment is slightly increased in diameter from the distal end to the proximal end.

The maximum outer diameter dimension R (see FIG. 2) of the distal tip 16 is set to 1.5 mm ≤ R ≤ 3.0 mm. With this configuration, the thrombus suction catheter 10 having the distal tip 16 can be used for thrombus suction therapy for blood vessels in the lower limbs, for example. Examples of blood vessels in the lower limbs include the iliac artery and vein, the femoral artery and vein, the popliteal (below-knee) artery and vein, and the like.

As shown in FIG. 10, the distal tip 16 includes a tube mounting groove 38 extending in the axial direction, which opens onto the lower surface. The tube mounting groove 38 extends across the entire axial length of the distal tip 16 with an approximately constant cross-sectional shape, and opens onto the distal face and proximal face of the distal tip 16. The tube mounting groove 38 has a groove inner surface with an arcuate cross section, and has an expanded shape whose width dimension increases downward. Here, the tube mounting groove 38 may have a cross-section of major-arc shape that exceeds half the circumference. With this configuration, the opening width of the tube mounting groove 38 may be made smaller than the diameter of the guide wire insertion tube 14, thereby making it easier to position the guide wire insertion tube 14 with respect to the distal tip 16.

The distal tip 16 has an X-ray transmission hole 40 that penetrates the bottom wall of the tube mounting groove 38 in the vertical direction. The X-ray transmission hole 40 is a long and narrow through hole that is elongated in the axial direction, and its distal end reaches an obstruction wall face 42 (described later) which is the wall face on the distal side of the distal lumen 34, while its proximal end reaches the proximal end of the distal tip 16. That is, the X-ray transmission hole 40 is provided continuously from the axially middle part to the proximal end of the distal tip 16, and opens toward the proximal end. **In** the present practical embodiment, the X-ray transmission hole 40 has a width dimension that is approximately constant from the distal end to the proximal end, but for example, the width dimension may increase from the distal end toward the proximal end.

A suction opening part 44 is formed in the large-diameter part 28 of the distal tip 16. The suction opening part 44 is formed by cutting out a portion in the circumferential direction of the peripheral wall of the distal lumen 34 in the large-diameter part 28, and as shown in FIG. 6, opens upward in the center portion in the left-right direction. The suction opening part 44 is provided at the distal end of the distal lumen 34, and the distal end of the distal lumen 34 opens to the lateral side (upward) on the outer peripheral surface of the distal tip 16 through the suction opening part 44, and does not extend to the distal side with respect to the suction opening part 44, with the distal side obstructed by the obstruction wall face 42. Therefore, in the present practical embodiment, the distal opening of the distal lumen 34 does not open toward the front, but opens only upward. The suction opening part 44 is preferably shaped so that it can be formed using a mold that is divided in the left-right direction during molding of the distal tip 16. In the present practical embodiment, the upper surface of the opening peripheral edge of the suction opening part 44 extends approximately parallel to the left-right direction.

The maximum depth dimension D of the suction opening part 44 is preferably set to 0.5 mm ≤ D ≤ 1.2 mm, and is set to 0.8 mm, for example. The axial length dimension L of the suction opening part 44 is preferably set to 5 mm ≤ L ≤ 20 mm, and is more preferably set to 8 mm ≤ L ≤ 15 mm. By setting the maximum depth dimension D and the axial length dimension L of the suction opening part 44 within the aforementioned ranges, it is possible to obtain a high suction force when suctioning blood containing a thrombus at a suction pressure of 600 mmHg, and it is also possible to suction even a large thrombus without clogging the suction opening part 44. Furthermore, the distal tip 16 can be made more compact in size.

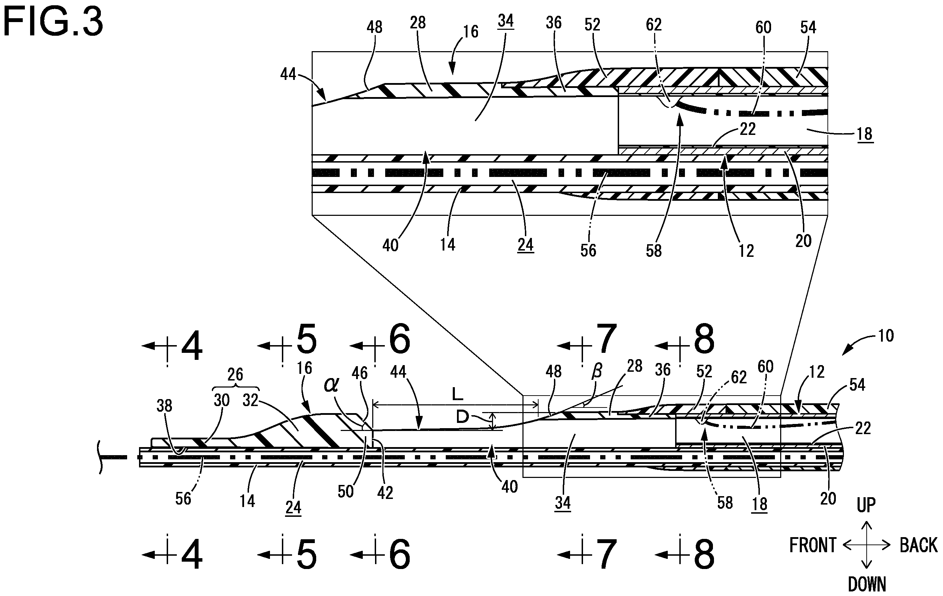

The inclination angle α (see FIG. 3) of a distal side wall face 46 of the suction opening part 44 with respect to the axial direction is larger than the inclination angle β (see FIG. 3) of a proximal side wall face 48 of the suction opening part 44 with respect to the axial direction. The inclination angle α of the distal side wall face 46 of the suction opening part 44 with respect to the axial direction is preferably set to 30 degrees ≤ α ≤ 75 degrees, and is set to approximately 45 degrees in the present practical embodiment. The obstruction wall face 42, which is continuous with the distal side wall face 46, extends in an approximately axis-perpendicular direction, and as shown in FIG. 3, a corner is formed at the boundary between the distal side wall face 46 and the obstruction wall face 42. On the other hand, the proximal side wall face 48 is smoothly continuous with the upper surface on the left and right sides of the opening peripheral edge of the suction opening part 44 without forming any corners. In the present practical embodiment, the distal side wall face 46 and the obstruction wall face 42 are both approximately flat surfaces, but they may be curved surfaces or have irregularities, for example.

In the present practical embodiment, the tube mounting groove 38 is formed so as to open downward on the outer peripheral surface of the distal tip 16, while the suction opening part 44 is formed so as to open upward on the outer peripheral surface of the distal tip 16. With this configuration, the tube mounting groove 38 and the suction opening part 44 are arranged at positions away from each other in the circumferential direction of the distal tip 16. The distal end portion of the X-ray transmission hole 40, which penetrates the bottom wall of the tube mounting groove 38, is approximately positioned in opposition to the suction opening part 44 in the diametrical direction of the distal lumen 34, and overlaps with the suction opening part 44 in the vertical direction. In the present practical embodiment, the width dimension of the suction opening part 44 is larger than the width dimension of the X-ray transmission hole 40. However, the suction opening part 44 and the X-ray transmission hole 40 may have approximately the same width dimension, or the X-ray transmission hole 40 may have a wider width than the suction opening part 44.

A thrombus crushing wall part 50 is provided between the small-diameter part 26 and the large-diameter part 28, and the proximal side end face of the thrombus crushing wall part 50 comprises the obstruction wall face 42 and the distal side wall face 46. The thrombus crushing wall part 50 has an axial thickness dimension larger than the thickness dimension of the peripheral wall of the distal lumen 34, and has large deformation rigidity. The tapered part 32 of the small-diameter part 26 is integrally and continuously provided at the distal side with respect to the thrombus crushing wall part 50, and the suction opening part 44 opens on the proximal side with respect to the thrombus crushing wall part 50.

The distal face of the suction tube 12 is abutted against the proximal face of the distal tip 16, and the guide wire insertion tube 14 is inserted into the tube mounting groove 38 of the distal tip 16. A cover tube 52 for welding is externally fitted over the suction tube 12, the guide wire insertion tube 14, and the thin-walled connection part 36 of the distal tip 16 so that the cover tube 52 axially straddles the region where the distal face of the suction tube 12 and the proximal face of the distal tip 16 are abutted against each other. After the cover tube 52 is heated and melted by a laser or the like, the cover tube 52 is hardened by cooling, whereby the suction tube 12, the guide wire insertion tube 14, and the distal tip 16 are mutually fixed by the cover tube 52. With this arrangement, the distal tip 16 is attached to the distal ends of the suction tube 12 and the guide wire insertion tube 14.

Here, the guide wire insertion tube 14 may be directly fixed to the distal tip 16 by means such as welding and bonding. In particular, since the guide wire insertion tube 14 extends significantly to the distal side with respect to the suction tube 12, it is desirable that the distal side part of the guide wire insertion tube 14 beyond the cover tube 52 be fixed to the distal tip 16 by means such as welding and bonding. Besides, the suction tube 12 and the guide wire insertion tube 14 are positioned with respect to each other by their proximal side parts beyond the cover tube 52 being inserted into a main tube 54. In the present practical embodiment, the main tube 54 and the cover tube 52 are formed of the same material as each other, and are integrated by the cover tube 52 being melted and then hardened. However, in the figures, a boundary line is illustrated in between to facilitate understanding.

By the distal tip 16 and the suction tube 12 being connected in a state where they are abutted against each other in the axial direction, the distal lumen 34 of the distal tip 16 and the suction lumen 18 of the suction tube 12 communicate with each other. The proximal opening of the distal lumen 34 and the distal opening of the suction lumen 18 have mutually different shapes and sizes, and in the state where the distal tip 16 and the suction tube 12 are abutted against each other, the proximal opening of the distal lumen 34 is partially open toward the proximal end on the left and right sides of the suction tube 12. The said open portion at the proximal opening of the distal lumen 34 is obstructed by the cover tube 52, which has been melted and deformed when the distal tip 16 and the suction tube 12 are joined by the cover tube 52, for example.

By the guide wire insertion tube 14 being inserted into and fixed to the tube mounting groove 38 of the distal tip 16, the X-ray transmission hole 40 penetrating the bottom wall of the tube mounting groove 38 is covered by the guide wire insertion tube 14. With this arrangement, the X-ray transmission hole 40, which penetrates a part of the peripheral wall of the distal lumen 34, is obstructed by the guide wire insertion tube 14, so that the negative pressure exerted on the distal lumen 34 is less likely to escape through the X-ray transmission hole 40, thereby efficiently applying suction force to the thrombus from the suction opening part 44. From the perspective of reliably obtaining the suction force, it is desirable that the X-ray transmission hole 40 be liquid-tightly obstructed by the guide wire insertion tube 14. However, it is acceptable as long as the opening of the X-ray transmission hole 40 is narrowed by the guide wire insertion tube 14 so that a thrombus of a size that needs to be removed cannot pass through the X-ray transmission hole 40. In the present practical embodiment, the X-ray transmission hole 40 is gradually widened from the distal end to the proximal end. Thus, the X-ray transmission hole 40 is obstructed by the guide wire insertion tube 14 in the portion where it overlaps with the suction opening part 44, while the X-ray transmission hole 40 is open on the left and right sides of the guide wire insertion tube 14 at the proximal side with respect to the suction opening part 44, and the said open portion is obstructed by the cover tube 52. However, the entire X-ray transmission hole 40 may be obstructed by the guide wire insertion tube 14.

In the thrombus suction catheter 10 according to the present practical embodiment constructed as above, a guide wire 56, which is shown hypothetically by the chain double-dashed line in FIG. 3, is inserted through the guide wire lumen 24 of the guide wire insertion tube 14. Then, the thrombus suction catheter 10 is moved within the blood vessel to the lesion site along the guide wire 56 that has been inserted into the blood vessel in advance. The distal tip 16 of the present practical embodiment includes the tapered small-diameter part 26 at its distal end portion, which enables excellent crossability with respect to the blood vessels and the like, and facilitates insertion to the lesion site. The position of the thrombus suction catheter 10 within the blood vessel can be grasped based on the length of the thrombus suction catheter 10, and can also be confirmed by imaging of the distal tip 16 with X-rays.

When the suction opening part 44 of the distal tip 16 has reached the lesion site where the thrombus exists, a negative pressure source such as a pump connected to the proximal side of the suction lumen 18 is used to drop the internal pressure of the distal lumen 34 and the suction lumen 18, and suction force is applied to the blood in the blood vessel from the suction opening part 44. This causes the blood containing the thrombus to be suctioned and removed from the blood vessel to the distal lumen 34 and the suction lumen 18, thereby treating the stenotic lesion caused by the thrombus. The thrombus and the blood retrieved in the suction lumen 18 will be discharged from the suction lumen 18 through a discharge line connected to the proximal side of the suction lumen 18.

The suction opening part 44 connected to the suction lumen 18 opens to the lateral side at the distal tip 16. This enables the suction force to be effectively applied to the lateral thrombus adhered to the blood vessel wall, and the thrombus can be efficiently suctioned and removed. In particular, the distal lumen 34 is obstructed by the obstruction wall face 42 on the distal side and does not open onto the distal face of the distal tip 16, so that the suction force can be concentratedly applied to the suction opening part 44. Besides, by operating the thrombus suction catheter 10 so that the distal tip 16 rotates within the blood vessel to change the direction of opening of the suction opening part 44 in the circumferential direction, it is also possible to suction and remove the thrombus adhered to the blood vessel wall over the entire circumference.

The distal tip 16 is formed separately from the suction tube 12 and the guide wire insertion tube 14, so that a large degree of freedom in designing its shape is achieved. This makes it possible to form the tapered small-diameter part 26 that can advantageously obtain ease of insertion into blood vessels, the suction opening part 44 that opens to the lateral side, and the like. In particular, the distal tip 16 of the present practical embodiment can be formed by molding such as injection molding, which increases the degree of freedom of its shape. Moreover, the distal tip 16 is not provided with a small-diameter guide wire lumen, which is difficult to form by molding, thereby avoiding the restriction on the degree of freedom of the shape of the distal tip 16 due to the small-diameter lumen. Therefore, it is possible to obtain the distal tip 16 according to the desired performance with a large degree of freedom of design, thereby achieving excellent thrombus suction performance and the like.

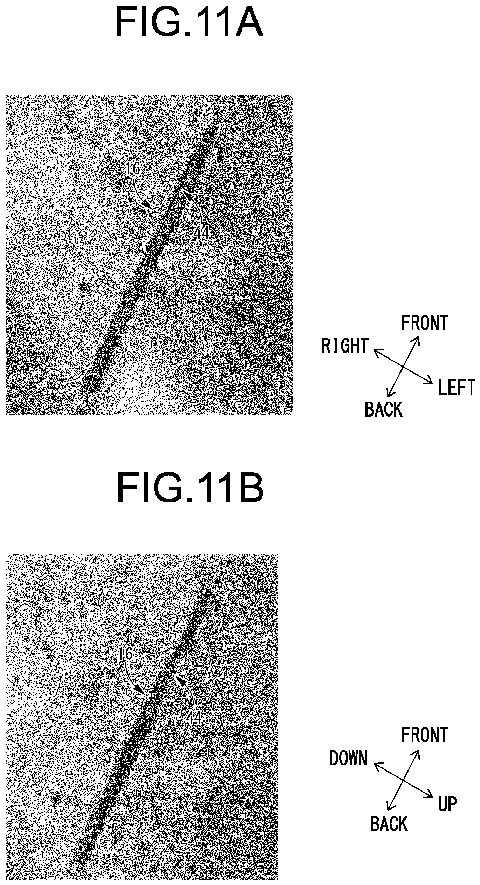

The direction of opening of the suction opening part 44 in the distal tip 16 can be confirmed relatively easily under the X-ray fluoroscopy. Specifically, when the direction of opening of the suction opening part 44 coincides with the X-ray fluoroscopy direction, in other words, when the X-ray fluoroscopy direction is the vertical direction of the thrombus suction catheter 10, the suction opening part 44 and the X-ray transmission hole 40 overlap with each other in the X-ray fluoroscopy direction. As a result, in the portion where the suction opening part 44 and the X-ray transmission hole 40 overlap with each other, the absorption of X-rays by the distal tip 16, which is made of X-ray-opaque resin, is suppressed, and the X-ray transmissivity is increased, so that the imaging of the distal tip 16 is faint at the suction opening part 44, as shown in FIG. 11A. Therefore, when there is a faint portion in the imaging of the distal tip 16 under the X-ray fluoroscopy, the suction opening part 44 opens to one side in the fluoroscopy direction. On the other hand, when the direction of opening of the suction opening part 44 and the X-ray fluoroscopy direction are mutually different, X-rays will not pass through the suction opening part 44 and the X-ray transmission hole 40, so that the imaging is dense, as shown in FIG. 11B. In this way, in the X-ray fluoroscopy, where it is difficult to obtain clear imaging, the direction of opening of the suction opening part 44 is distinguishable by the density of the imaging at the distal tip 16. This makes it easy to control the direction of opening of the suction opening part 44, thereby making it possible to exert suction force in the desired direction. Whereas the X-ray transmission hole 40 is covered by the guide wire insertion tube 14, since the guide wire insertion tube 14 has a higher X-ray transmissivity than that of the distal tip 16, it is less prone to affect the imaging under the X-ray fluoroscopy.

The distal tip 16 of the present practical embodiment includes the thrombus crushing wall part 50 on the distal side of the suction opening part 44. By moving the thrombus suction catheter 10 to the proximal side toward the thrombus with the thrombus crushing wall part 50 brought closer to the blood vessel wall, the thrombus crushing wall part 50 is brought into contact with the thrombus adhered to the blood vessel wall, and the thrombus can be scraped off from the blood vessel wall. This makes it possible to retrieve even those thrombi that are firmly adhered to the blood vessel wall and cannot be retrieved by suction force alone, thereby enhancing effect of thrombus suction therapy.

**In** the present practical embodiment in particular, in the thrombus crushing wall part 50, the distal side wall face 46, which is the contact surface with the thrombus, has a large inclination angle α with respect to the axial direction. Thus, the thrombus crushing wall part 50 has a shape that can effectively exert force on the thrombus adhered to the blood vessel wall by axial movement of the thrombus suction catheter 10 to the proximal side. This makes it possible to excellently obtain the effect of crushing the thrombus by the thrombus crushing wall part 50, thereby efficiently removing the thrombus adhered to the blood vessel wall.

While the distal side wall face 46 located on the distal side of the suction opening part 44 has a large inclination angle α with respect to the axial direction as described above, the proximal side wall face 48 located on the proximal side of the suction opening part 44 has the inclination angle β with respect to the axial direction that is smaller than the inclination angle α. With this configuration, when the thrombus suction catheter 10 is inserted into a blood vessel and pushed to the distal side, the proximal side wall face 48 is less likely to get caught on the inner circumferential surface of the blood vessel or the like, and the thrombus suction catheter 10 can be pushed smoothly.

Besides, since the thrombus crushing wall part 50 is thickest in the distal tip 16, it has excellent visibility under the X-ray fluoroscopy. The presence of such a thrombus crushing wall part 50 at the distal side of the suction opening part 44 makes it easy to grasp the distal end position of the suction opening part 44 under the X-ray fluoroscopy.

The thrombus suction catheter 10 may include a control mechanism 58 which is shown hypothetically by the chain double-dashed line in FIG. 3. The control mechanism 58 includes, for example, a control wire 60 inserted into the distal end of the suction tube 12 and a fixing part 62 provided on the inner circumferential surface of the distal end of the suction tube 12 to fix the distal end of the control wire 60 to the suction tube 12. By exerting the operating force, which has been input from the outside to the proximal side part of the control wire 60, on the suction tube 12 from the control wire 60, the control mechanism 58 can induce curving deformation of the distal end of the suction tube 12 and the distal end of the guide wire insertion tube 14. This makes it possible to change the direction of opening of the suction opening part 44 by the control mechanism 58, thereby efficiently applying the suction force to the thrombus. As the control mechanism 58, for example, the structure disclosed in International Publication No. WO 2018/061598 can be adopted, and a detailed explanation thereof is omitted here.

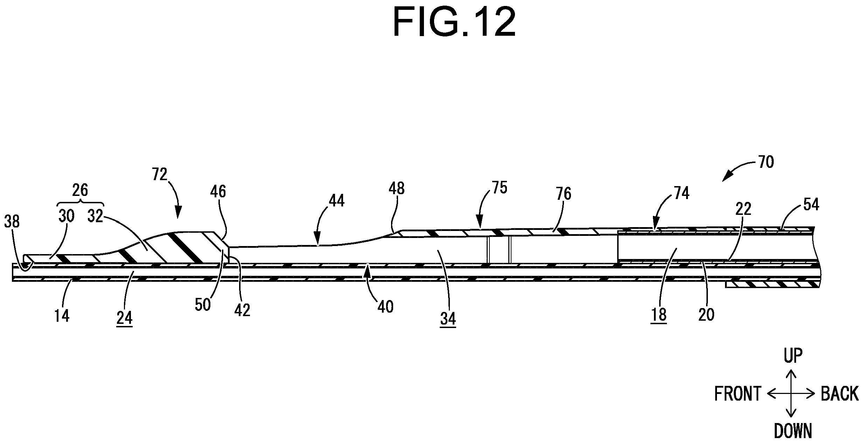

FIG. 12 shows a thrombus suction catheter 70 according to a second practical embodiment of the present invention. The thrombus suction catheter 70 has a structure in which a distal tip 72 is attached to the distal ends of a suction tube 74 and the guide wire insertion tube 14. **In** the following description, components and parts that are substantially identical with those in the first practical embodiment will be assigned like symbols and not described in any detail.

A large-diameter part 75 of the distal tip 72 has a longer axial length than the large-diameter part 28 of the first practical embodiment, and in particular, a tube connection part 76 extending to the proximal side is provided. The tube connection part 76 constitutes the proximal end of the large-diameter part 75, and is slightly larger in diameter than the distal side part. The large-diameter part 75 of the present practical embodiment does not include the thin-walled connection part 36 as in the first practical embodiment.

The suction tube 74 is inserted into the radial inside of the tube connection part 76 of the distal tip 72. Then, the tube connection part 76 and the suction tube 74 are mutually fixed by means such as welding and bonding, for example. The suction tube 74 of the present practical embodiment has a two-layered peripheral wall including the braided layer 20 comprising a braided body, and the inner layer 22 comprising a resin material and provided on the inside of the braided layer 20. The distal end of the suction tube 74, which is overlapped with the inner circumferential surface of the tube connection part 76, protrudes from the main tube 54 to the distal side, with the braided layer 20 exposed radially outward. The main tube 54 is positioned on the proximal side with respect to the tube connection part 76 of the distal tip 72, and the main tube 54 is not inserted into the tube connection part 76. Thus, a decrease in diameter of the tube connection part 76 and a sufficient inner diameter dimension of the suction tube 74 are both achieved.

As shown in the present practical embodiment, the connection structure between the suction tube and the distal tip is not limited to the structure using the cover tube 52 as in the first practical embodiment. According to the thrombus suction catheter 70 of the present practical embodiment, by obviating the cover tube 52, the number of parts can be reduced. Besides, the distal end of the suction tube 74 is inserted into the tube connection part 76 of the distal tip 72, so that the distal tip 72 and the suction tube 74 are easily positioned.

Furthermore, by increasing the inner diameter dimension of the distal tip 72 at the tube connection part 76, it is possible to prevent the distal tip 72 from becoming larger in diameter while allowing the suction tube 74 to become larger in diameter. **In** this case, if a step is formed on the distal side with respect to the tube connection part 76 on the inner circumferential surface of the distal tip 72, by the distal face of the suction tube 74 being abutted against the said step, the distal tip 72 and the suction tube 74 can be easily positioned in the axial direction.

While the present invention has been described in detail hereinabove in terms of the practical embodiments, the invention is not limited by the specific description thereof. For example, the forming material of the distal tip 16 is not limited to those exemplified in the preceding practical embodiment. Specifically, for example, the X-ray opaque material forming the distal tip 16 is not limited to the bismuth oxide exemplified in the preceding practical embodiment, and can be selected and adopted as appropriate from various known imaging agents, such as platinum, tungsten, bismuth subcarbonate, and barium sulfate.

The preceding practical embodiments exemplified the suction opening part 44 having a long-length opening shape that is elongated in the axial direction. However, the opening shape of the suction opening part 44 is not particularly limited, and for example, the axial dimension may be smaller than the circumferential width dimension, or the suction opening part 44 may be a spot-like shape such as a circle.

The preceding practical embodiments exemplified the structure including the suction opening part 44 that opens to the lateral side of the distal tip 16, but for example, the suction opening part connected to the distal side part of the suction lumen 18 may open toward the front of the distal tip 16. Besides, the suction opening part, which is the distal opening of the distal lumen 16, may be provided in plurality. For example, in addition to the suction opening part 44 that opens to the lateral side, a suction opening part that opens toward the front may also be provided. When there are a plurality of suction opening parts, for example, by allowing those suction opening parts to be selectively opened and closed, the suction force can be efficiently exerted.

Whereas the suction opening part 44 is preferably located on the opposite side in the diametrical direction to the tube mounting grooves 38, it is acceptable as long as the suction opening part 44 and the tube mounting grooves 38 are provided at mutually different locations in the circumferential direction of the distal tip 16. For example, the direction of opening of the suction opening part 44 and the direction of opening of the tube mounting groove 38 may be mutually shifted by approximately 90 degrees in the circumferential direction of the distal tip 16.

There is no need for the X-ray transmission hole 40 to reach the proximal end of the distal tip 16, and the X-ray transmission hole 40 could instead be provided so as to penetrate the axially middle part of the distal tip 16, for example.

The distal tip 16 preferably has a structure including the small-diameter part 26 and the large-diameter part 28 such that its distal end is smaller in diameter than its proximal end. However, it is not essential for the distal tip to include the small-diameter part and the large-diameter part, and for example, the outer diameter dimension of the distal tip may be approximately constant in the axial direction throughout.

The distal tip can also be formed by multicolor molding using two or more types of forming materials, for example. By partially varying the forming material of the distal tip in this way, it is possible, for example, to partially vary the X-ray transmissivity in the distal tip, making it easier to specify the direction of opening of the suction opening part when performing X-ray fluoroscopy of the distal tip, to reliably obtain both excellent flexibility of the catheter distal end and shape stability of the distal lumen 34, and the like.

### KEYS TO SYMBOLS

- 10: thrombus suction catheter (first practical embodiment)
- 12: suction tube
- 14: guide wire insertion tube
- 16: distal tip
- 18: suction lumen
- 20: braided layer
- 22: inner layer
- 24: guide wire lumen
- 26: small-diameter part
- 28: large-diameter part
- 30: semi-cylindrical part
- 32: tapered part
- 34: distal lumen
- 36: thin-walled connection part
- 38: tube mounting groove
- 40: X-ray transmission hole
- 42: obstruction wall face
- 44: suction opening part
- 46: distal side wall face
- 48: proximal side wall face
- 50: thrombus crushing wall part
- 52: cover tube
- 54: main tube
- 56: guide wire
- 58: control mechanism
- 60: control wire
- 62: fixing part
- 70: thrombus suction catheter (second practical embodiment)
- 72: distal tip
- 74: suction tube
- 75: large-diameter part
- 76: tube connection part

## Claims

1. A thrombus suction catheter comprising:
a suction tube including a suction lumen configured to suction a thrombus within a blood vessel;
a guide wire insertion tube including a guide wire lumen configured to allow a guide wire to be inserted; and
a distal tip attached to distal ends of the suction tube and the guide wire insertion tube, the distal tip including a distal lumen communicating with the suction lumen of the suction tube, wherein
a tube mounting groove extending in an axial direction is provided on an outer peripheral surface of the distal tip, and the guide wire insertion tube separate from the distal tip is inserted into the tube mounting groove and fixed to the distal tip.

2. The thrombus suction catheter according to claim 1, wherein
the distal lumen includes a suction opening part opening to a lateral side on the outer peripheral surface of the distal tip, and an obstruction wall face obstructing a distal side of the distal lumen is provided on the distal side of the suction opening part of the distal tip, and
the tube mounting groove is arranged at a position away from the suction opening part in a circumferential direction of the distal tip.

3. The thrombus suction catheter according to claim 2, wherein
a distal end portion of the distal tip comprises a small-diameter part including a tapered part, while a proximal end portion of the distal tip comprises a large-diameter part having a circular cylinder shape with a diameter larger than that of the small-diameter part, and
the suction opening part opens on the large-diameter part.

4. The thrombus suction catheter according to claim 2 or 3, wherein
the distal tip is formed of a resin material mixed with an X-ray opaque material, and
the distal tip includes an X-ray transmission hole penetrating a bottom wall of the tube mounting groove, the X-ray transmission hole being formed at a position that overlaps with the suction opening part in a diametrical direction of the distal tip.

5. The thrombus suction catheter according to claim 2 or 3, wherein a distal side wall face of the suction opening part has an inclination angle with respect to the axial direction of the distal tip larger than that of a proximal side wall face of the suction opening part.

6. The thrombus suction catheter according to claim 1 or 2, further comprising a control mechanism configured to induce curving deformation of the distal ends of the suction tube and the guide wire insertion tube by exerting operating force from an outside.
